# EUROPEAN PATENT APPLICATION

(11) **EP 1 980 626 A1**
(43) Date of publication of application: **15.10.2008**
(21) Application number: 07106144.4
(22) Date of filing: 13.04.2007
(51) Int. Cl.: C12Q 1/68

(54) **Involvement of lipid kinase, and signal transduction pathway comprising said lipid kinase, in resistance to HER2-targeting therapy**

(71) Applicant: Het Nederlands Kanker Instituut, 1066 CX Amsterdam (NL)
(72) Inventor: Berns, Katrien, 2061 LX Bloemendaal (NL); Bernards, René, 1391 AD Abcoude (NL)
(74) Representative: van Loon, C.J.J.

(57) **Abstract**

The invention is related to a method for determining whether an individual suffering from cancer is at risk of exhibiting or acquiring a reduced response towards HER2-targeting therapy. In one aspect, the invention utilizes the activity of a signal transduction pathway modulated by a lipid kinase for determining said risk.

## Description

The invention relates methods for determining whether an individual suffering from cancer is at risk of exhibiting or acquiring a reduced response towards HER2-targeting therapy, and the use of inhibitors of lipid kinase activity for increasing sensitivity towards HER2-targeting therapy.

Trastuzumab (Herceptin®) is a monoclonal antibody that targets the Human Epidermal growth factor Receptor 2 (HER2), a receptor tyrosine kinase which is over-expressed in 20-25% of all invasive breast cancers. Striking initial responses are observed in combination with chemotherapy in approximately 60% of patients. However, the majority of responding patients eventually develop resistance to trastuzumab-based therapies (Slamon, et al., New Engl J Med 344, 783-792 (2001); Vogel, et al. J Clin Oncol 20, 719-726 (2002); Cobleigh, et al. J Clin Oncol 17, 2639-2648 (1999)).

The epidermal growth factor (EGF) family of receptor tyrosine kinases consists of four receptors, ErbB1 (HER1), ErbB2 (HER2/Neu), ErbB3 (HER3), and ErbB4 (HER4). Members of the EGF-receptor family contain an extracellular domain that is involved in ligand binding and receptor dimerization, a single transmembrane domain and a cytoplasmic tyrosine kinase domain. Ligand binding induces dimerization of a receptor, resulting in autophosphorylation of the intracellular domains which creates docking sites on the receptor for signal transducing molecules. These signal transducing molecules comprise Grb-2, which activates Ras and a Ras-dependent mitogen activated protein kinase signaling cascade ultimately resulting in activation of transcription factors such as c-fos, AP-1, and Elk-1 that promote gene expression and contribute to cell proliferation; PLC, leading to an increase in intracellular Ca2+ and activation of PKC; phosphatidylinositol 3-kinase (P13K), resulting in the localized production of PIP3 [phophatitidylinositol (3,4,5)-triphosphatel, which subsequently recruits AKT to the plasma membrane where it is phosphorylated and activated; and JAK/STAT, resulting in nuclear gene expression activation through activation of the JAK/STAT pathway.

After binding of a ligand, the EGF receptor is rapidly internalized by endocytosis. This process is thought to require clathrin and occurs in clathrin-coated pits, which pinch off from the plasma membrane to form vesicles that move to the early endosome. From the early endosome, receptors can either be recycled back to the cell surface, or they can move through the late endosome to the lysosome for proteolytic degradation.

The mechanism by which trastuzumab exerts its anti-tumor activity is not fully understood. Trastuzumab has been suggested to induce antibody-dependent cellular cytotoxicity (ADCC); to inhibit HER2 extracellular domain cleavage; and to inhibit PI3K/AKT survival signaling, either by downregulating HER2 signaling or by increasing Phosphatase and tensin homologue deleted on chromosome TEN (PTEN) membrane localization and phosphatase activity, leading to a decline in PI3K/AKT pathway activation and inhibition of proliferation (Morris and Carey, Oncology 20, 1763-1771 (2006)).

The mechanism by which cells become resistant towards trastuzumab is presently unknown. Possible mechanisms include activation of HER2-related receptors which take over the role of HER2 in said cell such as HER1 and HER3, or non-HER receptors, such as insulin-like growth factor I receptor; mutation of HER2 preventing binding of trastuzumab; blockage of binding of trastuzumab by increased cell surface mucin; increased degradation of HER2; downregulation of p27KIP, a cell cycle inhibitor; and loss of PTEN activity, a lipid phosphatase ((Sergina, et al. Nature 445, 437-441 (2007); Morris and Carrey, Oncology 20:1763-1771 (2006)).

Although any of the mechanisms mentioned above might be involved in trastuzumab resistance, they can not explain all of the observed HER2-resistant tumors. Half of the breast cancer patients that over-express HER2 are initially non-responsive to trastuzumab-based therapy, while the majority of the patients become resistant to trastuzumab during treatment. An understanding of the resistance mechanisms would stimulate the development of rational drug combinations to circumvent resistance and allow selection of patients that are likely to respond.

In the present invention, it was found that mutation of PI3K, or activation of a signal transduction pathway involving PI3K, correlates with the occurrence of HER2 resistance.

The invention therefore provides a method for determining whether an individual suffering from cancer is at risk of exhibiting or acquiring a reduced response towards HER2-targeting therapy, said method comprising determining the nucleotide sequence of PI3K, or a part thereof, in a cell sample derived from said cancer of said individual, comparing said sequence with the corresponding sequence of PI3K in a reference sample, and determining said risk on the basis of the comparison.

A cell sample refers to a relevant sample of said individual comprising cancer cells or remnants of cancer cells such as nucleic acid molecules or amino acid molecules. A relevant cell sample comprises cells or remnants of cells comprising nucleic acid molecules or proteins that are derived from a cancerous cell.

PI3-kinases comprise a family of enzymes that phosphorylate phosphatidylinositol at the 3' position of the inositol ring, generating phosphatidylinositol 3,4,5-triphosphate (PI3,4,5-P3) which is thought to act as a second messenger that controls cellular activities and properties including proliferation, survival, motility and morphology. Phospho-inositol derivatives can function as docking sites by interacting with proteins that comprise a pleckstrin homology (PH) domain, thereby regulating the localization and often also the activity of PH-comprising proteins. Class I PI3-kinase family members comprise heterodimers of a regulatory subunit termed p85, and a catalytic subunit termed p110. Mutations in the gene encoding the regulatory subunit p85α were found in some primary colon and ovarian tumours (Bader et al. Nature Reviews Cancer 5: 921-929 (2005). Four catalytic subunits are known to date, including PIK3Catalytic Alpha (PIK3CA), PIK3CB, PIK3C2B, and PIK3CG. Of these, the gene encoding PIK3CA has been associated with cancer. Somatic mutations in the PIK3CA gene have been identified in colon, breast, liver, brain, stomach, lung, and ovary tumor samples (Karakas et al. British Journal of Cancer 94: 455-459 (2006)), while overexpression of the gene has been associated with ovarian cancer, cervical cancer, and head and neck squamous cell carcinoma (Pedrero et al. Int. J. Cancer 114: 242-248 (2005)). Some mutations have been identified at high frequency within both the helical and catalytic kinase domains (Bader et al. Nature Reviews Cancer 5: 921-929 (2005).

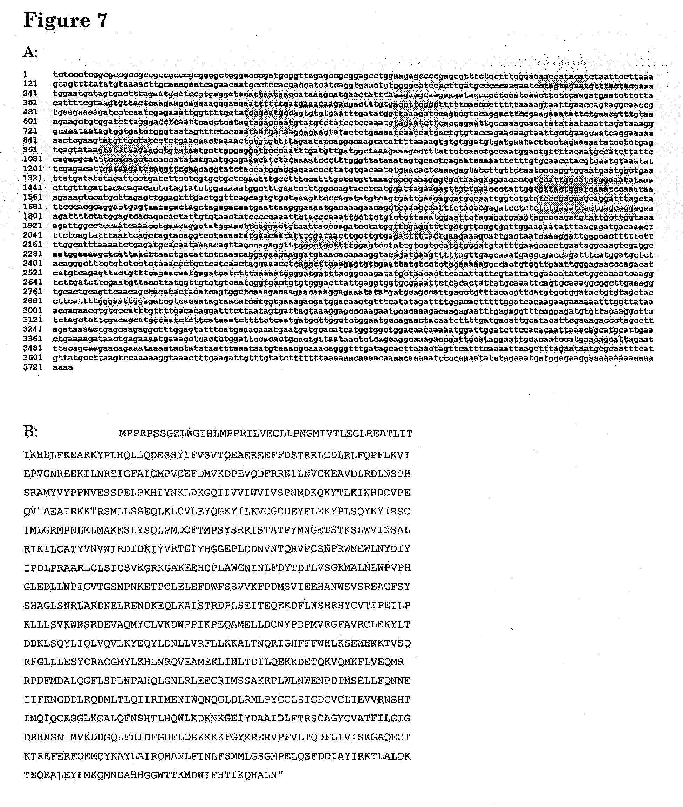

In a preferred embodiment, therefore, a method according to the invention comprises determining the nucleotide sequence of PIK3CA, or a part or derivative thereof. It is preferred that said part or derivative thereof comprises nucleic acid sequences that encode one or more of the amino acid residues listed in Table 4. It is further preferred that said part or derivative thereof comprises nucleic acid sequences that encode the C-terminal half of the protein, comprising a helical domain and a kinase domain, which starts at about amino acid residue number 520 (see Figure 7). In an even further preferred embodiment, said part or derivative thereof comprises nucleic acid sequences that are present in exon 9 or exon 20, which encode parts of the helical and catalytic domain, respectively.

A sequence of PIK3CA in a reference sample refers to the wild type nucleotide sequence of PIK3CA (see Figure 7). As a model for the wild type sequence, reference is made to GenBank accession number NM_006218. However, a reference sample can also be derived from a non-affected individual such as, for example, a non-affected close relative.

A nucleotide sequence of PIK3CA that is altered compared to a nucleotide sequence of PIK3CA in a reference sample refers to any altered nucleotide sequence that results in an alteration of the amino acid sequence of the encoded protein. It is preferred that said alteration results in an enhanced activity of a signal transduction pathway involving PIK3CA.

Alterations that have been identified in PIK3CA in human cancers, and that can enhance an activity of PIK3CA comprise alteration of any of the amino acids at position 38 (arginine), 60 (glutamine), 88 (arginine), 104 (proline), 106 (glycine), 108 (arginine), 110 (glutamic acid), 111 (lysine), 118 (glycine), 122 (glycine), 124 (proline), 345 (asparagine), 350 (aspartic acid), 378 (cysteine), 405 (serine), 418 (glutamic acid), 420 (cysteine), 453 (glutamic acid), 539 (proline), 542 (glutamic acid), 545 (glutamic acid), 661 (glutamine), 701 (histidine), 733 (lysine), 901 (cysteine), 909 (fenylalanine), 1008 (serine), 1011 (proline), 1021 (tyrosine), 1025 (threonine), 1035 (glutamic acid), 1043 (methionine), 1044 (asparagine), 1046 (alanine), 1047 (histidine), 1049 (glycine), or 1065 (histidine), whereby the numbering relates to the PIK3CA amino acid sequence as depicted in Figure 7 (see Table 4). Therefore, the presence of one of more of these alterations is indicative of an enhanced risk of resistance towards HER2-targeting therapy,

In a preferred embodiment, a method according to the invention comprises determining a nucleotide sequence from PIK3CA at any of positions 1624, 1633, 1634, 3075, 3127, 3140, or 3147 of a nucleic acid encoding PIK3CA, as depicted in Figure 7, or of the corresponding position in a genomic nucleic acid molecule encoding PIK3CA, or of the corresponding position in any part or derivative of the indicated nucleotide sequence, whereby an alteration of the encoded amino acid at position 542 (glutamic acid), 545 (glutamic acid), 1025 (tyrosine), 1043 (methionine), 1047 (histidine), or 1049 (glycine), from the indicated amino acid to another amino acid, is indicative of an enhanced risk of resistance towards HER2-targeting therapy.

In a further preferred embodiment, a method according to the invention comprises determining a nucleotide sequence from PIK3CA at any of positions 1624, 1633, 1634, or 3140 of a nucleic acid encoding PIK3CA, as depicted in Figure 7, or of the corresponding position in a genomic nucleic acid molecule encoding PIK3CA, or of the corresponding position in any part or derivative of the indicated nucleotide sequence, whereby an alteration of the encoded amino acid at position 542 (glutamic acid), 545 (glutamic acid), and 1047 (histidine), from the indicated amino acid to another amino acid, is indicative of an enhanced risk of resistance towards HER2-targeting therapy.

A nucleotide sequence of PIK3CA can be determined by any method known in the art, including but not limited to sequence analysis of a genomic region encoding PIK3CA and sequence analysis of a mRNA product or a derivative of a mRNA product such as a cDNA product, by any method known in the art, including but not limited to dideoxy sequencing, matrix-assisted laser desorption/ionization time-of-flight mass spectrometry, and sequencing by hybridization, including hybridization with sequence-specific oligonucleotides and hybridization to oligonucleotide arrays. The nucleotide sequence of PIK3CA can also be determined by application of mutation analysis methods such as single stranded conformation polymorphism, DNA heteroduplex analysis, denaturing gradient gel electrophoresis and thermal gradient gel electrophoresis.

Sequence analyses can be performed either by direct sequence analysis of a relevant nucleic acid molecule comprising the PIK3CA gene or a mRNA product thereof, or by indirect sequencing of a relevant nucleic acid after amplification of all or any part of the PIK3CA gene or a mRNA product thereof. Alternative direct or indirect methods comprise hybridization protection assay, allele-specific amplification, ligase-mediated detection, primer extension, and restriction fragment length analysis.

In an alternative embodiment, the presence of a mutation in PIK3CA that is indicative of an enhanced risk of resistance towards HER2-targeting therapy can be determined by analysis of the encoded protein by, for example, protein sequence determination, two dimensional gel electrophoresis, multidimensional protein identification technology, ELISA, liquid chromatography-mass spectrometry (LC-MS), matrix-assisted laser desorption/ionization time-of-flight mass spectrometry (MALDI-TOF), or the use of antibodies that interact with either a non-mutated normal form of PIK3CA, or with a mutated variant form of PIK3CA.

In another aspect, the invention provides a method for determining whether an individual suffering from cancer is at risk of exhibiting or acquiring a reduced response towards HER2-targeting therapy, said method comprising determining a (the) status of activity of a signal transduction pathway involving PI3K in a cell sample derived from said cancer of said individual, and determining said risk on the basis of said status.

In a preferred method according to the invention, said status of activity is compared to the status of activity of said signal transduction pathway in a reference sample, and said risk is determined on the basis of said comparison.

The term signal transduction refers to a process by which a cell converts an extracellular signal to a response, comprising the relay of a signal by conversion from one physical or chemical form to another. A signal transduction pathway is often composed of a series of signals that are transmitted from one compartment of a cell, such as for example the membrane, to another compartment, such as for example the nucleus. A signal transduction pathway may involve distinct signals, such as, for example, specific second messengers such as cellular calcium concentration and cyclic adenosine monophosphate concentration, specific ions that enter or exit a cell through ion channels, protein modification such as phosphorylation or dephosphorylation, protein localization, protein cleavage, protein degradation, protein activation by for example binding of co-factors such as guanosine triphosphate, lipid modification such as lipid cleavage, lipid phosphorylation and lipid dephosphorylation, and transcriptional activation. The result of activation of a signal transduction pathway can be diverse and depends, for example, on the type of cell, the history of a cell, and other signal transduction pathways that are active in said cell. Activation of a signal transduction pathway can result in cell proliferation, cell activation, cell differentiation, cell remodelling, and cell death, amongst other effects.

A status of activity of a signal transduction pathway involving PI3K can be determined by quantifying an indicator of said activity. Said quantifiable indicator comprises amplification of a gene encoding PI3K, a level of expression of PI3K, an activity of PI3K, and a level of expression of at least one downstream indicator gene for which the level of expression depends on the activity of a signal transduction pathway involving PI3K.

A reference sample is used for comparison to determine whether a transduction pathway involving PIK3CA is affected relative to said reference sample. Said reference sample may comprise a reference cell sample. As will be clear, a reference cell sample comprises breast cells, or remnants thereof, if the diseased individual suffered from breast cancer. A reference sample comprises prostate cells, or remnants thereof, if the diseased individual suffered from prostate cancer. Said reference sample can be assayed together with said sample from said individual.

It is preferred that relevant data from a reference sample are stored on a storage device and compared to the status of activity of a pathway involving PIK3CA in said individual. A storage device indicated any device on which data can be stored, including but not limited to a lab note book, a computer readable storage medium, and a data base comprising relevant data from patients of which the status of activity of a pathway involving PIK3CA was determined and that received HER2-targeting therapy.

An individual suffering from cancer is at risk of exhibiting or acquiring a reduced response if the status of activity of said pathway in the diseased individual is similar to the status of activity of said pathway in a reference sample, if said reference sample is obtained from a patient that showed no response or a poor response or a patient that became resistant upon treatment with HER2-targeting therapy.

If said reference sample is derived from a healthy person or a person that showed a good response upon treatment with HER2-targeting therapy, a similarity in the status of activity of the pathway between said individual and said reference sample is indicative of a reduced risk of exhibiting or acquiring a reduced response towards HER2-targeting therapy. It will be clear to a person skilled in the art, that a risk can also be classified on the basis of differences in the status of activity of said pathway in said individual compared to a reference sample.

To determine whether a status of activity of a pathway involving PIK3CA is similar to the status of activity of said pathway in a reference sample, a threshold level can be determined. A sample that scores above the pre-determined threshold is classified as having a reduced risk of exhibiting or acquiring a reduced response towards HER2-targeting therapy if said reference sample is derived from a healthy person or a person that showed a good response upon treatment with HER2-targeting therapy, while a sample that scores below said threshold is classified as having an increased risk.

A transduction pathway involving PIK3CA refers to one or more signal transduction pathways that are affected by activation or inhibition of PIK3CA. Inhibition or activation of said signal transduction pathway can result in differences in activity of gene products, differences in localization of gene products, differences in levels of expression of gene products, and differences in modification of gene products.

The status of activity of a pathway involving PIK3CA can be determined by any means known in the art, including but not limited to determining the amplification status of the PIK3CA gene, determining the level of expression of a gene product from the PIK3CA gene or of other genes in said pathway, and determining the activity of a gene product from the PIK3CA gene.

In this aspect of the invention, a preferred cell sample represents a quantitative copy of all genes that are expressed at the time of collection of the sample. To preserve a quantitative copy, samples can be processed in numerous ways, as is known to a skilled person. Preferably, they are freshly prepared from cells or tissues at the moment of harvesting, or they prepared from surgical biopsies that are stored at -70°C until processed for sample preparation. Alternatively, tissues or surgical biopsies are stored under protective conditions that preserve the quality of the RNA. Preferred examples of these preservative conditions are fixation using e.g. formaline, RNase inhibitors such as RNAsin (Pharmingen) or RNasecure (Ambion), or RNalater (Ambion).

In an alternative embodiment, the cell sample is prepared from a needle aspiration biopsy, which is a procedure by which a thin needle is inserted in a tissue to extract cells. A needle aspiration biopsy can be processed and stored under protective conditions that preserve the quality of the RNA. Examples of these preservative conditions are fixation using e.g. formaline, RNase inhibitors such as RNAsin (Pharmingen) or RNasecure (Ambion), or RNalater (Ambion).

A preferred method for determining a status of activity of a pathway involving PIK3CA comprises determining amplification of a gene encoding PIK3CA.

Genomic amplification of PIK3CA has been detected in several cancers, including gastric cancer (Byun et al. 2003. Int J Cancer 104:318-27) ovarian cancer (Campbell et al. 2004. Cancer Res. 64: 7678-7681), and oral squamous cell carcinoma (Kozaki et al. 2006. Cancer Sci. 97: 1351-8), and is associated with increased expression of PIK3CA transcript.

An amplification status of the PIK3CA gene comprises fluorescent in situ hybridization, chromogenic in situ hybridization, Southern blotting, hybridization of total genomic DNA to a microarray, and Quantitative Polymerase Chain Reaction (qPCR).

In another preferred embodiment, said status of activity of a pathway involving PIK3CA is determined by determining a level of expression of a gene product from the PIK3CA gene.

Amplification or transcriptional activation can lead to overexpression of PIK3CA, leading to enhanced activity of a pathway involving PIK3CA. The level of expression of a protein product from the PIK3CA gene can be determined by any means known in the art, including but not limited to two-dimensional gel electrophoresis, enzyme linked immunosorbent assay (ELISA), and Western blotting.

Methods to determine the nucleic acid levels of expression of the PIK3CA gene are known to a skilled person and include, but are not limited to, Northern blotting, quantitative PCR, and microarray analysis.

Northern blotting comprises the quantification of the nucleic acid expression product from the PIK3CA gene by hybridizing a labeled probe that specifically interacts with said nucleic acid expression product, after separation of nucleic acid expression products by gel electrophoreses. Quantification of the labeled probe that has interacted with said nucleic acid expression product serves as a measure for determining the level of expression. The determined level of expression can be normalized for differences in the total amounts of nucleic acid expression products between two separate samples by comparing the level of expression of a gene that is known not to differ in expression level between samples.

Quantitative-PCR (qPCR) provides an alternative method to quantify the level of expression of nucleic acid products from the PIK3CA gene. Following a reverse transcriptase reaction, qPCR can be performed by real-time PCR (rtPCR), in which the amount of product is monitored during the reaction, or by end-point measurements, in which the amount of a final product is determined. As is known to a skilled person, rtPCR can be performed by either the use of a nucleic acid intercalator, such as for example ethidium bromide or SYBR® Green I dye, which interacts which all generated double stranded products resulting in an increase in fluorescence during amplification, or by the use of labeled probes that react specifically with the generated double stranded product of the gene of interest. Alternative detection methods that can be used are provided by dendrimer signal amplification, hybridization signal amplification, and molecular beacons.

Different amplification methods, known to a skilled artisan, can be employed for qPCR, including but not limited to PCR, rolling circle amplification, nucleic acid sequence-based amplification, transcription mediated amplification, and linear RNA amplification.

Microarray analyses comprise the use of selected biomolecules that are immobilized on a surface. A microarray usually comprises nucleic acid molecules, termed probes, which are able to hybridize to nucleic acid expression products such as a nucleic acid expression product from the PIK3CA gene. The probes are exposed to labeled sample nucleic acid, hybridized, and the abundance of nucleic acid expression products in the sample that are complementary to a probe is determined. The probes on a microarray may comprise DNA sequences, RNA sequences, or copolymer sequences of DNA and RNA. The probes may also comprise DNA and/or RNA analogues such as, for example, nucleotide analogues or peptide nucleic acid molecules (PNA), or combinations thereof. The sequences of the probes may comprise fragments of genomic DNA. The sequences may also be synthetic nucleotide sequences, such as synthetic oligonucleotide sequences.

In yet a further preferred embodiment, determining a status of activity of a signal transduction pathway involving PIK3CA comprises determining a kinase activity of PIK3CA.

Overexpression or mutation of PIK3CA can result in enhanced kinase activity of PIK3CA, conferring resistance towards HER2-targeting therapy. Suitable assays for determining kinase activity of PIK3CA, such as those based on the conversion of phosphoinositol (4,5) biphosphate to phosphoinositol (3,4,5) triphosphate, are known in the art and include PI3 kinase ELISA (Echelon Biosciences Inc), TruLight® Phosphoinositide 3-Kinase Assay (Merck/Calbiochem), and PI 3-Kinase HTRF® assay (Upstate/Millipore).

In a further preferred method according to the invention, determining a status of activity of a signal transduction pathway involving PIK3CA comprises determining a nucleotide sequence from PIK3CA. A nucleotide sequence of PIK3CA that is altered compared to a nucleotide sequence of PIK3CA in a reference sample, whereby it is preferred that said alteration results in an enhanced activity of a signal transduction pathway involving PIK3CA.

Preferred are alterations at any of positions 1624, 1633, 1634, or 3140 of a nucleic acid encoding PIK3CA, as depicted in Figure 7, or of the corresponding position in a genomic nucleic acid molecule encoding PIK3CA, or of the corresponding position in any part or derivative of the indicated nucleotide sequence, whereby an alteration of the encoded amino acid at position 542 (glutamic acid), 545 (glutamic acid), and 1047 (histidine), from the indicated amino acid to another amino acid, is indicative of an enhanced risk of resistance towards HER2-targeting therapy.

In yet another preferred embodiment, determining a status of activity of a signal transduction pathway involving PIK3CA comprises determining a level of expression of at least one PIK3CA-indicator gene.

A PIK3CA-indicator gene is a gene of which the level of expression is controlled by a pathway involving PIK3CA. A PIK3CA-indicator gene can be identified by comparing the levels of expression of genes that are expressed in cells with different activities of a pathway involving PIK3CA, as estimated by one of the methods described in this application. The level of expression of said indicator gene can be up-regulated or down-regulated upon activation of a pathway involving PIK3CA.

Differences in activity of a pathway involving PIK3CA are obtained, for example, by inhibiting PIK3CA activity by contacting cells with inhibitors of PIK3CA, or by activating PIK3CA activity by exogenous expression of PIK3CA or activated mutants of PIK3CA in cells. Known inhibitors of PIK3CA comprise wortmannin, LY294002, PX-866, ZSTK474, and expression of anti-sense RNA molecules, siRNA molecules or an antibody against one of the subunits of PIK3CA.

It is preferred that said at least one PIK3CA-indicator gene is identified by comparing levels of expression of genes in cells that express wildtype PIK3CA with levels of expression of said genes in cells that express an altered PIK3CA, whereby said altered PIK3CA comprises an alteration that results in activation of said protein.
Supervised analysis can be used to identify a PIK3CA-indicator gene of which the level of expression is indicative of activity of a pathway involving PIK3CA. Said PIK3CA-indicator gene can be validated by comparison of the level of expression of said PIK3CA-indicator gene in samples from patients that respond to HER2-targeting therapy and samples from patients that were resistant or acquired resistance to HER2-targeting therapy.

For the simultaneous detection of multiple nucleic acid gene expression products, qPCR methods such as reverse transcriptase- multiplex ligation-dependent amplification (rtMLPA), which accurately quantifies up to 45 transcripts of interest in a one-tube assay (Eldering et al., Nucleic Acids Res 2003; 31: e153), and microarray analyses can be employed, as is or will be known to a skilled person.

In yet another aspect, a method for determining whether an individual suffering from cancer is at risk of exhibiting or acquiring a reduced response towards HER2-targeting therapy according to the invention, refers to a therapy selected from gene therapy, chemo-therapy, compound-mediated therapy, siRNA-mediated therapy, protein therapy, and antibody-mediated therapy.

Said HER2-targeting therapy is preferably selected from tyrosine kinase inhibitors such as lapatinib ditosylate (Tykerb®; GSK), Tak 165 (Takeda Pharmaceuticals), gefitinib (Iressa®, Astra Zeneca), erlotinib (OSI-774, Tarceva), CP-724714 (Pfizer), and CI1033 (PD183805; Pfizer); antisense techniques, ribozymes, and siRNA which down-modulate the level of expression of HER2; antibodies, such as trastuzumab (Herceptin®), pertuzumab (Omnitarg®); and vaccines such as recombinant dHER2 vaccine (GSK) and Neuvenge® (APC8024; Dendreon)..

In a preferred embodiment, said HER2-targeting therapy comprises antibody-mediated HER2-targeting therapy.

Antibodies that bind to the extracellular domain of ErbB2 (HER2/Neu) can block the function of HER2 overexpression. Currently, two antibodies are known that bind to different epitopes in the extracellular domain of HER2. The recombinant humanized HER2 monoclonal antibody pertuzumab (Genentech, San Francisco, CA) sterically blocks dimerization of HER2 with EGFR and HER3, while binding of trastuzumab might block activation of HER2 by promoting receptor endocytosis (Nahta et al. 2006. Nat Clin Pract Oncol. 3: 269-280).

In a more preferred embodiment, said antibody-mediated therapy comprises trastuzumab.

Trastuzumab (Herceptin) is the first humanized antibody approved for the treatment of HER2-positive metastatic breast cancer. Trastuzumab, in combination with paclitaxel, is indicated for treatment of patients with metastatic breast cancer whose tumors overexpress the HER2 protein. Resistance to trastuzumab, however, is a common problem that ultimately culminates in treatment failure. Identification of patients that are or might become resistant to trastuzumab is important for adequate treatment of these patients.

In another aspect, the invention provides the use of an inhibitor of PIK3CA, or preferably an inhibitor of a mutant of PIK3CA, in the preparation of a medicament for the treatment of a HER2-resistant cancer patient.

Activation of PIK3CA, or a pathway involving PIK3CA, results in resistance to HER2-targeting therapy. Therefore, inhibition of PIK3CA activity might restore sensitivity towards HER2-targeting therapy. Known inhibitors of PIK3CA comprise wortmannin, LY294002, PX-866, ZSTK474, anti-sense RNA molecules, siRNA molecules or an antibody against one of the subunits of PI3kinase, such as the P85 or P110 subunit.

Malignant cells that are known to express HER2, and that benefit or that might benefit from HER2-targeting therapy, comprise prostate cells, bladder cells, ureter cells, breast cells, bone cells, colon cells, gastro-oesophagal cells, kidney cells, liver cells, ovarian cells, pancreatic cells, squamous lung cells, and lung adenocarcinoma cells. Clinical trials comprising HER2-targeting therapy are ongoing for patients suffering from osteosarcoma, and cancers of the lung, pancreas, salivary gland, colon, prostate, endometrium and bladder.

In a preferred embodiment, said cancer patient is a breast cancer patient.

HER2-positive breast cancers tend to be more aggressive than other types of breast cancer, and are less responsive to hormone treatment. Sensitizing, or re-sensitizing HER2-positive breast cancer cells towards HER2-targeting therapy will improve the outcome of this type of cancer.

### Legends to the Figures

Figure 1
   shRNA barcode screen identifies PTEN as modulator of Trastuzumab efficacy.
Figure 2
   PTEN downregulation and active PI3K signaling confers Trastuzumab resistance in cell culture.
Figure 3
   Kaplan-Meier survival curves for Trastuzumab-treated HER2 positive patients.
Figure 4
   PTEN immunohistochemical analysis.
Figure 5
   PIK3CA sequence analysis.
Figure 6
   Kaplan-Meier survival curves for Trastuzumab-treated HER2 positive patients for separate cohort studies.
Figure 7
   Nucleotide and protein sequence of wild type PIK3CA, as provided by GenBank accession number NM_006218.

### Examples

### Example 1

### Methods

### Cell culture, transfection and retroviral infection

The human breast cancer cell lines BT-474 and SKBR-3 were purchased from the American Type Culture Collection (ATCC, Manassas) and were cultured in Dulbecco's Modified Eagle Medium (DMEM) supplemented with 8% heat-inactivated fetal calf serum, penicillin and streptomycin. For both the BT-474 and SKBR-3 cell lines, subclones were generated that ectopically express the murine ecotropic receptor. Ecotropic retroviral supernatants were produced by transfection of Phoenix packaging cells. Transfections were performed with the calcium phosphate precipitation technique. Viral supernatants were filtered through a 0,45 µm filter and infections were performed in the presence of 8 µg/ml polybrene (Sigma). Drug selections in BT-474 and SKBR-3 cells were performed with 2 µg/ml puromycin. Trastuzumab was obtained from the NKI/AVL hospital pharmacy, dissolved in MQ, and a 20 mg/ml stock was stored at -20 °C.

### shRNA bar code screen

BT-474 cells were infected with retroviruses representing the complete NKi RNAi library as described previously (Berns et al. 2004. Nature 428: 431-437). Infected cells were selected with puromycin (2.0 µg/ml) and plated into two populations at low density. One population was left untreated, while the other population was cultured in 10 µg/ml Trastuzumab. During the screen, cells were trypsinized and replated, to remove small cell clumbs. After 4 weeks in culture the treated and untreated populations were collected. Genomic DNA was isolated with the use of DNAzol (Life Technologies). The shRNA inserts were amplified from genomic DNA by PCR using the primers pRS-T7-fw, 5'GGCCAGTGAATTGTAATACGACTCACTATAGGGAGGCGGCCCTTG AACCTCCTCGTTCGACC-3', containing a T7 RNA polymerase promoter sequence, and pRS-8-rev, 5'-TAAAGCGCATGCTCCAGACT-3'. Purified PCR products were used for linear RNA amplification, and purified RNA probes were labeled with cyanine-3 (Cy3) or cyanine-5 (Cy5) fluorescent groups (Kreatech). Labeled RNA probes from untreated and Trastuzumab-treated cells were combined and hybridized to oligonucleotide arrays as described (Berns et al. 2004. Nature 428: 431-437). Quantification of the resulting fluorescent images was performed with Imagene 5.6 (BioDiscovery), local background was subtracted, and the data were normalized and 2log transformed.

### Plasmids

The retroviral vector expressing a constitutive active mutant of PIK3CA (110aCaaX) and the knock down vector for PTEN have been described previously (Kortlever et al. 2006. Nature Cell Biol. 8: 877-884). The PIK3CA (wt) and PIK3CA (H1047R) cDNAs were generated with PCR using the caPIK3CA as a template and cloned into the pMXiresGFP retroviral vector. Control infections were performed with a GFP expressing retrovirus or with a hairpin targeting GFP.

### Patients

For the NKI/AVL cohort, patients were eligible based on HER2 overexpression by immunohistochemistry (IHC 3+) and/or HER2 gene amplification by chromogenic in situ hybridization (CISH). 34 patients with HER2-overexpressing primary breast carcinomas who subsequently developed metastatic breast cancer and received either Trastuzumab monotherapy (n=3), Trastuzumab plus taxane (n=8), Trastuzumab plus vinorelbine (n=16), Trastuzumab plus vinorelbine and lonafarnib (n=5), Trastuzamab with paclitaxal and carboplatin (n=1) or adjuvant Trastuzumab (n=1), were collected from the Netherlands Cancer Institute/Antoni v Leeuwenhoek hospital and surrounding hospitals. The treatment started between September 2002 and September 2005. Data on relapse-free survival (defined as the time to progression) were available for all 34 patients. Twenty-six of the tumours had high nuclear grade III, seven had moderate nuclear grade II and one tumour had low nuclear grade. ER status was analyzed by IHC and positive in 14 and negative in 19 tumours, for one tumour it was not possible to retrieve the ER status. Formalin-fixed paraffin embedded archival material was used for IHC, genomic DNA isolation, PCR and sequence analysis. For the MD Anderson cohort, primary tumour material from 21 patients with breast cancer that was confirmed as HER2 amplified (by FISH and/or 3+ positivity on IHC) was obtained from the frozen breast tissue tumour bank at M. D. Anderson Cancer Center under the auspices of an IRB-approved protocol. Seventeen of the tumours had high nuclear grade (III by Modified Black) and ER status by IHC was positive in 12 and negative in 9 tumours. All patients were treated with a Trastuzumab-based regimen for metastatic disease; Trastuzumab monotherapy (n=3) or Trastuzumab plus taxane (n=8) or Trastuzumab plus vinorelbine (n=6) or Trastuzumab plus other chemotherapy (n=4). In all cases, the treatment regimen utilized for this analysis was the first Trastuzumab-containing regimen administered to each patient for metastatic disease. Time to progression was calculated for all patients starting from the first Trastuzumab-based regimen that was administered.

Average age at diagnosis was 46.6 years and ranged from 25 to 74.1 (NKI: 47.8, 28.9-74.1, MD Anderson: 44.5, 25-65). Average time to progression was 11.8 and ranged from 0.7 to 44.7 months (NKI: 11.3, 0.7-37.5, MD Anderson: 12.6, 0.8-44.7). During follow-up, 48 events occurred and 7 patients were censored (NKI: 29 and 5, MD Anderson: 19 and 2). The prevalence of PIK3CA mutation was 25% (NKI: 21%, MD Anderson: 33%). Low PTEN expression was observed in 25% of the patients.

### DNA isolation

Genomic DNA was isolated from ten 10 µm-thick paraffin embedded tissue sections. Sections were de-paraffinated twice for 5 min in xylene, rehydrated in 100%, 96% and 70% ethanol for 30 s each, stained with haematoxylin for 30 s, rinsed with water and incubated overnight in 1 M NaSCN at 37 °C to remove crosslinks. Slides were rinsed twice 10 min in PBS at room temperature, and completely air-dried. Tumour tissue was scraped from the glass with a scalpel to obtain at least 70% tumour cells (as indicated by an experienced breast cancer pathologist on a hematoxylin and eosin stained slide) in 200 ml Qiagen ATL buffer (QIAamp DNA extraction kit), transferred to eppendorf tubes and incubated with 27 µl proteinase-K (protK 15 mg/ml stock) at 450 rpm at 55 °C. Two more aliquots of 27µl protK were added at 20 and 28 h. After a total protK incubation of around 44 h, DNA isolation proceeded as in the manufacturer's protocol (Qiagen, Cat.51306). Genomic DNA from frozen tissue was isolated using a standard phenol-chloroform protocol.

### PIK3CA PCR, sequencing, and mutational analysis

The primers we used for PCR and sequencing were as follows; for exon 9-forward; 5'-AGTAACAGACTAGCTAGAGACAAT-3', exon 9-reverse; 5'-GAGATCAGCCAAATTCAGTTATTTT-3', exon 20-forward; 5'-CAGGAGATGTGTTACAAGGCTTAT-3', exon 20-reverse;

5'-TCAGTTCAATGCATGCTGTTTAAT-3'. PCR reactions were performed on 10-100 ng of genomic DNA. After an initial denaturation step of 94 °C for 3 minutes, 30 cycles of amplification were performed with denaturation at 94 °C for 30 seconds , annealing at 55 °C for 30 seconds and extension at 72 °C for 1 minute ; followed by a final extension cycle of 72 °C for 10 minutes. PCR products were purified over a QIAquick spin column (Qiagen) and were sequenced using the BigDye Terminator Cycle Sequencing Kit (Applied Biosystems) and an ABI 3730 automated capillary sequencer. For all PCR products with sequence variants both forward and reverse sequence reactions were repeated for confirmation.

### PIK3CA SNP analysis

A SNP-based approach was used to detect common PIK3CA mutations. We used a Sequenom (San Diego, CA) MALDI TOF MassArray system. DNA around the known potential PIK3CA mutation hotspot sites 111, 542, 545 and 1047 was first amplified and a primer extension reaction was run to determine the potential SNP base. Both the polymerase chain reaction (PCR) primers and the extension primers were designed using the Sequenom Assay Design software. This program allows for muliplex reactions of up to 30 different SNPs per well. The initial PCR reactions were done in a 384 well format according to manufactures' instructions and the PCR reactions were cleaned up using EXO-SAP (also supplied by Sequenom). The primer extension reactions were done using Sequenom's IPLEX chemistry and according to their protocol. The IPLEX reactions were then desalted using Sequenom's Clean Resin and spotted onto Spectrochip matrix chips using a Samsung Nanodispenser. The chips were then run on the Sequenom MassArray. Sequenom Typer Software was used to interpret the mass spectra that were generated and to report the SNPs based on expected masses. All spectra generated were run in duplicate and were visually inspected.

### Reverse phase protein lysate array (RPPA)

Lysis buffer (1% Triton X-100, 50mm HEPES, pH 7.4, 150mM NaCl, 1.5mM MgCl2, 1mM EGTA, 100mM NaF, 10mM Na Pyrophosphate, 1mM Na3VO4, 10% glycerol, 1mM PMSF and ^{~}img/ml aprotinin) was used to lyse 21 macrodissected fresh frozen metastatic HER2 amplified human breast tumours by homogenization. The protein lysates (supernatants) were diluted to 1 mg/ml, boiled with 1% SDS and diluted in five twofold serial dilutions with additional lysis buffer using a Tecan liquid handling robot. A robotic GeneTAC arrayer (Genomic Solutions, Inc., Ann Arbor, MI) created 1152 spot, 192 sample arrays on a nitrocellulose-coated glass slide (FAST Slides, Schleicher & Schuell BioScience, Inc. USA, Keene, NH) that included the serial dilutions of each HER2 amplified sample (Liang et al. 2007. Nature Cell Biol 9: 218-224; Tibes et al. 2006. Mol Canc Ther 5: 2512-2521; Sheehan et al. 2005. Mol Cell Proteom 4: 346-355). This arrayed slide was probed with a validated primary antibody to PTEN (Cell Signaling, Inc.) and the signal was amplified using a DakoCytomation (Carpinteria, CA) catalyzed system (CSA). A secondary antibody (anti-rabbit) was used as a starting point for signal amplification. The stained slide was scanned, analyzed, and quantitated using Microvigene software (VigeneTech Inc., North Billerica, MA) to generate a serial dilution-signal intensity supercurve for PTEN from all samples on the slide and each sample was then fitted to this supercurve to generate logarithmic values representative of relative PTEN signal intensity for each sample lysate. The expression of PTEN was corrected for protein loading using the average expression levels of 50 other probed proteins in each sample. Across the 21 samples, the tumours with the bottom 25% in terms of quantified PTEN expression were classified as having low PTEN expression, which the other 75% of tumours were classified as having high expression of PTEN.

### Immunohistochemistry

Serial sections of 3 µm from the paraffin blocks were de-paraffinated in xylene, and hydrated in a graded series of alcohol. Staining was performed using the Lab Vision Immunohistochemical Autostainer (Lab Vision Corporation, Fremont, CA, USA) with primary antibodies towards PTEN (DAKO, 1:200), HER2 (clone 3B5, 1:3000; van de Vijver et al. 1988. New Engl J Med 319: 1239-1245), and ER (estrogen receptor-a (ER; 1D5+6F11, dilution 1:50, Neomarkers, Lab Vision Corporation, Fremont, CA, USA). Detection was performed with antigen retrieval method (citrate pH 6.0).

### Scoring

The PTEN expression level was scored semi-quantitatively based on staining intensity and distribution using the immunoreactive score (IRS) as described elsewhere (Chui et al. 1996. Br J Cancer 73: 1233-1236; Friedrichs et al. 1993. Cancer 72: 3641-3647). Briefly, IRS = SI (staining intensity) x PP (percentage of positive cells). SI was assigned as: 0= negative; 1=weak; 2=moderate; 3=strong. PP is defined as 0=0%; 1=0-25%; 2=25-50%; 3=50-75%; 4=75-100%. Vascular endothelium known to express normal PTEN was used as positive control. Based on the IRS score, PTEN status was graded as follows: low PTEN expression, IRS 0-3; high PTEN expression, IRS 4-12. For one tumour it was not possible to retrieve the PTEN score. Stainings for ER were interpreted as negative when no tumour cells were stained, and as positive when more than 10 % tumour cell showed staining of ER in the nuclei. Her2neu staining was scored as negative when no (score 0), less than 10% (score 1) or greater than 10% (score 2) of the tumour cells showed a weak staining, and as positive when greater than 10% of the tumour cells showed a strong membrane-staining (score 3). In case a tumour showed a score of 2+ for HER2 IHC, we performed chromogenic in situ hybridization (CISH) for HER2. For the assessment of the gene status by CISH, samples with an average of at least or more than six copies per nucleus were considered to be HER2 amplified. CISH was performed using the Spot-Light CISH Polymer Detection Kit (Zymed, San Francisco, CA, USA) as described (Hannemann et al. 2006. Br J Cancer 95: 1334-1341).

### Statistical analysis

We evaluated the association between time to progression and two candidate risk factors PIK3CA mutation and PTEN expression using multivariate Cox regression with age as the time scale. Follow-up started at the age of diagnosis and ended at the age of progression, the age at death or the age at censoring, whichever came first. PTEN expression was measured differently at the two participating centers. For categorization of the continuous expression values into low and high, we chose a commonly used cut point for the NKI measurements (range 0-12, cut point <3 vs >3, which resulted in 25% of NKI patients being low) and used the corresponding percentile of the expression values among MD Anderson patients as cut point for the MD Anderson patients (range 57.3-488.4, cut point <105 vs > 105). PTEN expression was missing for one subject from NKI. All analyses were stratified by center and adjusted for age by using this variable as the time scale. Confounding was further evaluated for grade and ER status. Kaplan-Meier plots were produced in order to graphically illustrate the event history by the two candidate risk factors. Log-rank tests were performed to evaluate the homogeneity of group-specific survival curves. We evaluated the association between treatment response (complete response (CR) or partial remission (PR)/ stable disease (SD) > 6 months versus progressive disease (PD) or partial remission (PR)/ stable disease (SD) < 6 months) and PIK3CA mutation and PTEN expression using Fisher's exact test. All tests were two-sided.

### Results

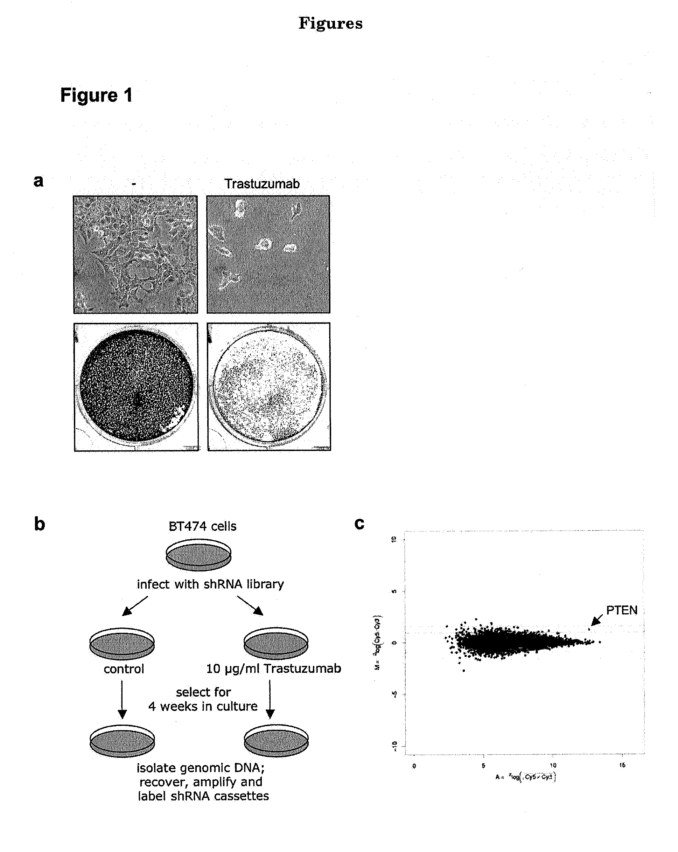

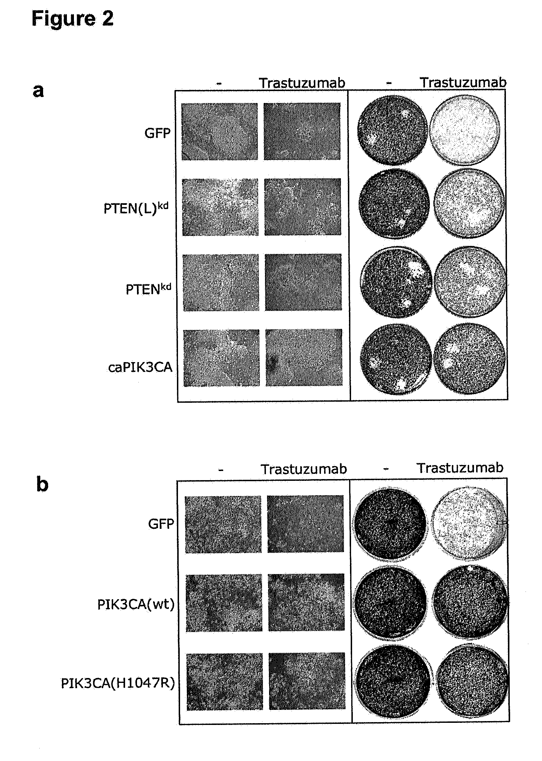

As an unbiased approach to identify genes involved in Trastuzumab resistance, we used a large-scale RNA interference genetic screen in the HER2-overexpressing breast cancer cell line BT-474. We have previously described the generation of a library of 24,000 shRNA retroviral vectors targeting some 8,000 human genes for suppression by RNA interference as well as a technology to rapidly screen such libraries, named siRNA bar code screening (Brummelkamp et al. 2006. Nat Chem Biol 2: 202-206; Berns et al. 2004. Nature 428: 431-437). In short, this technology allows one to identify shRNAs that are enriched in a population based on the relative abundance of a 'bar code" identifier (a unique 19-mer DNA sequence) in the vector, which is measured on a DNA micro-array that carries the 24,000 different bar code sequences. BT-474 cells respond to Trastuzumab predominantly by a reduction in proliferation rate rather than apoptosis or complete proliferation arrest (Fig 1a). To identify genes whose suppression by shRNA cause resistance to Trastuzumab, BT-474 cells were infected with the shRNA library and selected for the presence of the shRNA vectors with puromycin. After selection, cells were split into two populations and plated at low density. One population was left untreated and was used as a reference while the other was exposed to 10 µg/ml Trastuzumab. After 4 weeks, cells were harvested, genomic DNA isolated and shRNA cassettes were recovered by PCR amplification and hybridized to DNA microarrays as described (Berns et al. 2004. Nature 428: 431-437; see Fig 1b). We combined the data from five independent Trastuzumab barcode screens and analyzed the relative abundance of the recovered shRNAs. Fig 1c shows the relative abundance of the shRNA vectors in the Trastuzumab treated population as compared to the untreated population. We selected the top 5 shRNA vectors that were enriched by Trastuzumab selection, of which the shRNA targeting the PTEN tumour suppressor gene was most prominently enriched (marked with an arrow). When tested in second round selection, only the vector targeting PTEN conferred resistance to Trastuzumab. Importantly, a second, independent shRNA knocking down PTEN expression (Kortlever et al. 2006. Nature Cell Biol 8: 877-884) also conferred resistance to Trastuzumab, effectively ruling out the possibility that "off target" effects of the shRNA vectors caused the resistance phenotype (Fig. 2a). Our finding that knockdown of PTEN in BT-474 cells decreases sensitivity to Trastuzumab is consistent with earlier findings which demonstrated that PTEN loss is associated with resistance to Trastuzumab-based therapy (Nagata et al. 2004. Cancer Cell 6: 117-127). Importantly, our observation that of the 8,000 genes tested, only knock down of PTEN conferred resistance to Trastuzumab suggests that the PTEN pathway plays a dominant role in Trastuzumab resistance. However, since loss of PTEN is only observed in a fraction of breast cancers, it is unlikely that loss of PTEN alone explains the frequent primary and acquired non-responsiveness to Trastuzumab observed in the clinic.

Activating mutations in the gene encoding the p 1 10a catalytic subunit of PI3K (PIK3CA) have been identified in some 25% of primary breast cancers (Saal et al. 2005. Cancer Res 65: 2554-2559). The majority of these mutations reside in two hotspots in exon 9 and 20 and it has been demonstrated that the two most common mutations (E545K and H1047R,) result in increased PI3K pathway signaling (Isakoff et al. 2005. Cancer Res 65: 10992-11000).
To test whether activation of the PI3K pathway results in Trastuzumab resistance, we retrovirally transduced BT-474 cells with a constitutively active mutant of PIK3CA; caPIK3CA (p110αCaaX). Figure 2a shows that expression of this mutant rendered BT-474 almost completely insensitive towards Trastuzumab. Furthermore, expression of PIK3CA(wt) and the breast cancer-derived mutant PIK3CA(H1047R) also conferred resistance to Trastuzumab in SK-BR3 cells (Fig. 2b) and in BT-474 cells (data not shown). Apparently, a small increase in PI3K signaling through overexpression of PIK3CA (wt) is sufficient to counteract the growth inhibitory effects of Trastuzumab in cell culture. These findings are consistent with a major role of the PI3K pathway in the development of resistance to Trastuzumab.

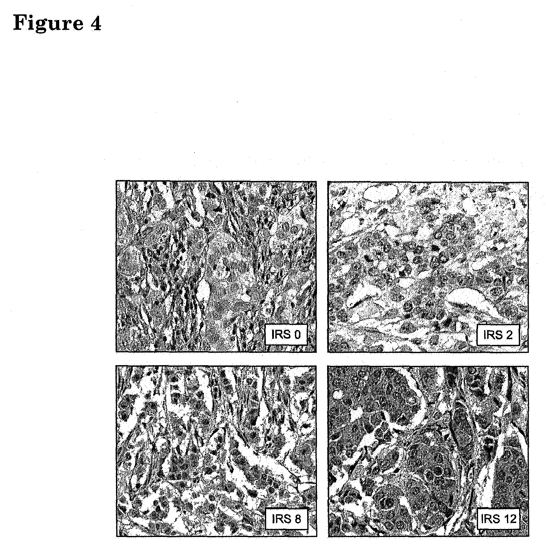

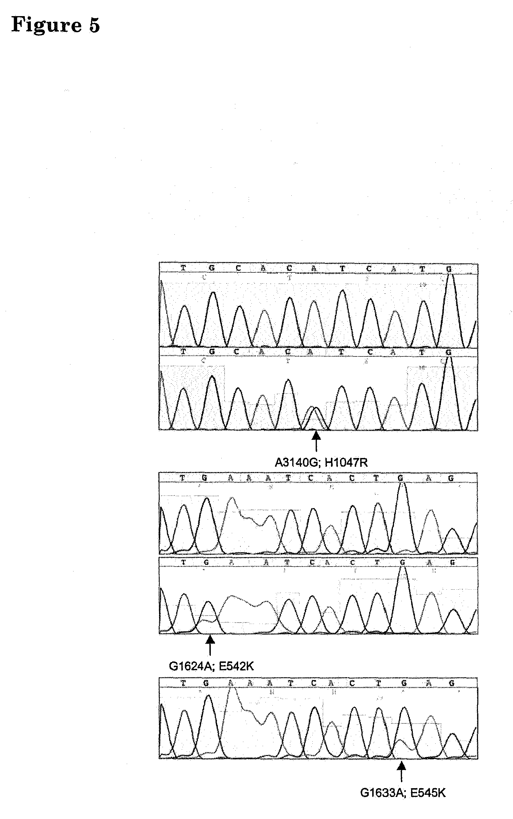

The high frequency of activating mutations in PIK3CA (around 25%; Saal et al. 2005. Cancer Res 65: 2554-2559) in breast cancer, together with our observation that activated PI3K signaling induces strong Trastuzumab resistance in cell culture, led us to investigate whether cancer-associated mutations of PIK3CA or altered levels of PTEN were able to predict Trastuzumab resistance in the clinic. For this, we made use of samples from two series of HER2 over-expressing patients with metastatic breast cancer treated at the Antoni van Leeuwenhoek hospital (n=34) and the MD Anderson Cancer Center (n=21). These 55 patients received Trastuzumab monotherapy (n=6), or Trastuzumab in combination with a chemotherapy regimen (n=49). Both the PTEN expression levels (using either immunohistochemical analysis or protein arrays) and the PIK3CA mutation status (by direct sequencing or SNP based analysis) were evaluated in this study and correlated to the therapeutic response to Trastuzumab-based therapy (see methods and Figures 4 and 5).

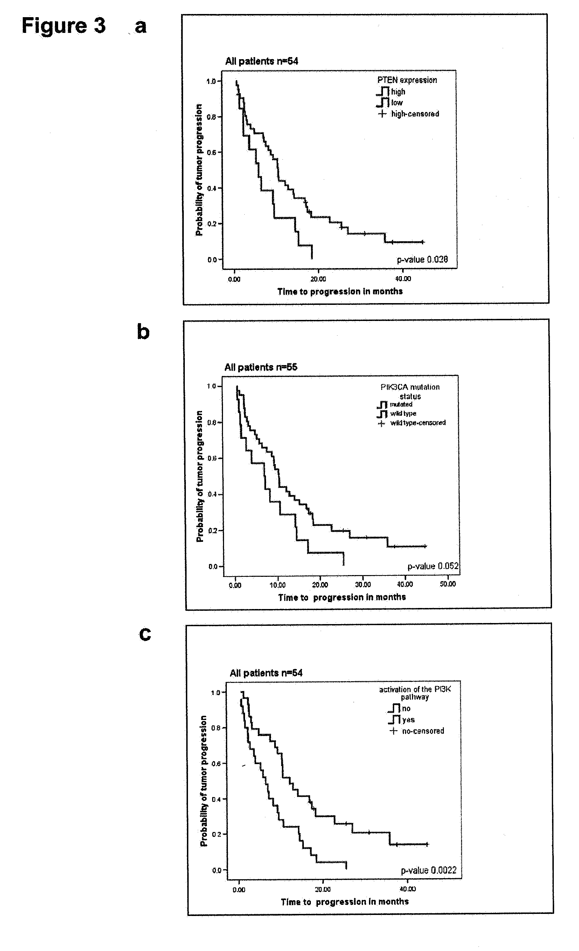

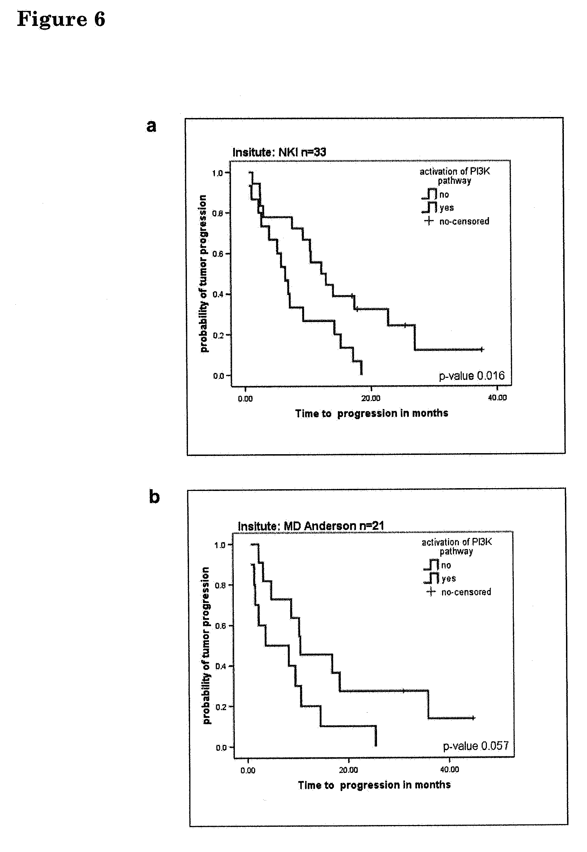

We observed reduced PTEN expression in 24% (13 out of 54; one sample could not be scored) of the tumours examined (Table 2). Kaplan-Meier survival curves were generated based on clinical follow-up data on time to progression after initiation of the Trastuzumab-based treatment. Figure 3a demonstrates that patients with PTEN low tumours have a statistically significantly (p=0.028) poorer relapse-free survival. Subsequent PIK3CA sequence analysis of the 55 tumour samples identified 10 mutations in exon 20 (H1047R) and 4 in exon 9 (E542K and E545K) corresponding to a PIK3CA mutation frequency of 25%, in agreement with the published frequency of PIK3CA mutations in breast cancer (Saal et al. 2005. Cancer Res 65: 2554-2559; see Table 2). Interestingly, 12 of 14 of the PIK3CA mutations were identified in PTEN high tumours, which is in agreement with the finding that PTEN loss and PIK3CA mutation are rarely present in the same tumour in breast cancer (Saal et al. 2005. Cancer Res 65: 2554-2559). Apparently, abrogation of either PTEN expression or oncogenic PIK3CA mutation relieves the selective pressure to target the other. We then determined whether activation of the PI3K pathway by oncogenic PIK3CA mutation would predict Trastuzumab-based treatment outcome. The Kaplan-Meier survival curve in Figure 3b indeed demonstrates a borderline significantly (p=0.052) poorer relapse-free survival among patients with mutation-positive tumours. However, the PIK3CA wild type tumours are contaminated with 28% (11 out of 40) PTEN low tumours, which are associated with shorter time to disease progression (Fig. 3a). Since PTEN loss and PIK3CA mutation both contribute to PI3K pathway activation, we classified the patients in two groups having either "activated" PI3K pathway (PTEN low + PIK3CA mutants; n=25) and "not-activated" PI3K pathway (PTEN high + PIK3CA wt; n=29) and generated Kaplan-Meier survival plots. The curves shown in Figure 3c demonstrate that patients with either PTEN loss or PIK3CA mutation have a significantly worse relapse-free survival following Trastuzumab-based treatment than patients without PTEN loss or PIK3CA mutation (p=0.002). Importantly, the significance of the relapse-free survival difference between the two groups was more marked when both events (PTEN loss and PIK3CA mutation) were considered. Analysis of the two cohorts separately gave very similar survival curves (Figure 6). Hazard ratios based on multivariate Cox regression analysis with age as time scale, stratified for center and adjusted for ER status, indicate that PI3K pathway status is an independent significant risk factor for disease progression (HR 2.1, p=0.02; Table 1). Furthermore, bad clinical responses (defined as no response at all, or partial remission and stable disease shorter than 6 months) correlated significantly with activation of a PI3K pathway (Fisher's exact test p=0.049), but not with PTEN loss or PIK3CA mutation independently (Table 3). These data suggest that combined PTEN expression and PIK3CA hotspot mutation analysis may serve as an important predictor for Trastuzumab-based therapy response. There was no evidence of heterogeneity of the effects of PTEN and PIK3CA between the two centers (p>0.47).

These data provide the first evidence that PIK3CA mutations contribute to unresponsiveness to Trastuzumab (Fig. 3b), and combining PTEN status and PIK3CA status identified twice as large a group of patients at increased risk for relapse (46%) compared to PTEN alone (24%, Fig. 3c). Our data therefore indicate that assessment of both PIK3CA mutation
status and PTEN expression level, reflecting a pathway activation status, is required for optimal prediction of Trastuzumab responsiveness in HER2 amplified breast tumours. In addition, the present study highlights the central importance of PI3K signalling in Trastuzumab responsiveness, which in turn suggests combination therapeutic strategies to treat Trastuzumab unresponsive breast cancer or to prevent emergence of resistance.

### Tables

**Table 1. Multivariate Cox regression analysis**

| Individual and joint effects of PTEN expression and PIK3CA mutation on time to progression | | | | |
|---|---|---|---|---|
| | *events* | *HR* | *95% Cl* | *P* |
| PTEN high | 35 | 1.0 | | |
| PTEN low | 13 | 2.0 | 0.9-4.1 | 0.0714 |
| | | | | |
| PIK3CA wt | 34 | 1.0 | | |
| PiK3CA mut | 14 | 1.6 | 0.8-3.4 | 0.195 |
| | | | | |
| Not-activated P13K pathway | 23 | 1.0 | | |
| Activated P13K pathway | 25 | 2.1 | 1.1-3.9 | 0.0237 |

| | | | | |
|---|---|---|---|---|
| HR, hazard ratio; wt, wildtype; mut, mutant; Cl, confidence interval; not-activated P13K pathway, PTEN high + P1K3CA wt; activated PI3K pathway, PTEN low + PIK3CA mutant. HRs based on Cox regression with age as time scale, stratified for center and adjusted for ER status | | | | |

**Table 2. PIK3CA mutation in HER2 positive patients n=55**

| | | *PIK3CA* | |
|---|---|---|---|
| | | no mutation | mutation |
| PTEN | low | 11 | 2 |
| | high | 29 | 12 |
| | missing (n=1) | | |
| | | | |
| Histological Grade | well diff | 1 | 0 |
| | moderate diff | 9 | 1 |
| | poorly diff | 30 | 13 |
| | missing (n=1) | | |
| | | | |
| ESR1 | neg | 21 | 7 |
| | pos | 19 | 7 |
| | missing (n=1) | | |

**Table 3. Cross-tables therapy response**

| Fisher's exact test p=0.194 | | **PTEN loss** | | |
|---|---|---|---|---|
| | | *no* | *yes* | *total* |
| PD or PR/SD< 6 months response | no | 13 | 7 | 20 |
| CR or PR/SD> 6 months response | | 28 | 6 | 34 |
| total | | 41 | 13 | 54 |
| | | | | |

| Fisher's exact test p=0.335 | | **PIK3CA mutation** | | |
|---|---|---|---|---|
| | | *no* | *yes* | *total* |
| PD or PR/SD< 6 months response | no | 13 | 7 | 20 |
| CR or PR/SD> 6 months response | | 28 | 7 | 35 |
| total | | 41 | 14 | 55 |
| | | | | |

| Fisher's exact test p=0.049 | | **activation of the PI3K pathway** | | |
|---|---|---|---|---|
| | | *no* | *yes* | *total* |
| PD or PR/SD< 6 months | no response | 7 | 13 | 20 |
| CR or PR/SD> 6 months | response | 22 | 12 | 34 |
| total | | 29 | 25 | 54 |

| | | | | |
|---|---|---|---|---|
| PD: progressive disease, CR: complete remission, PR: partial remission, SD: stable disease | | | | |

**Table 4**

| Amino acid residues of PIK3CA that are frequently altered | |
|---|---|
| 38 (arginine) | 542 (glutamic acid) |
| 60 (glutamine) | 545 (glutamic acid) |
| 88 (arginine) | 661 (glutamine) |
| 104 (proline) | 701 (histidine) |
| 106 (glycine) | 733 (lysine) |
| 108 (arginine) | 901 (cysteine) |
| 110 (glutamic acid) | 909 (fenylalanine) |
| 111 (lysine) | 1008 (serine) |
| 118 (glycine) | 1011 (proline) |
| 122 (glycine) | 1021 (tyrosine) |
| 124 (proline) | 1025 (threonine) |
| 345 (asparagine) | 1035 (glutamic acid) |
| 350 (aspartic acid) | 1043 (methionine) |
| 378 (cysteine) | 1044 (asparagine) |
| 405 (serine) | 1046 (alanine) |
| 418 (glutamic acid) | 1047 (histidine) |
| 420 (cysteine) | 1049 (glycine) |
| 453 (glutamic acid) | 1065 (histidine), |
| 539 (proline) | |

## Claims

1. A method for determining whether an individual suffering from cancer is at risk of exhibiting or acquiring a reduced response towards HER2-targeting therapy, said method comprising
a. determining the nucleotide sequence of PI3K, or a part thereof, in a cell sample derived from said cancer of said individual;
b. comparing said sequence with the corresponding sequence of PI3K in a reference sample; and
c. determining said risk on the basis of the comparison.

2. A method for determining whether an individual suffering from cancer is at risk of exhibiting or acquiring a reduced response towards HER2-targeting therapy, said method comprising
a. determining a status of activity of a signal transduction pathway involving PI3K in a cell sample derived from said cancer of said individual;
b. determining said risk on the basis of said status.

3. Method according to claim 2, wherein said status of activity is compared to the status of activity of said signal transduction pathway in a reference sample, and said risk is determined on the basis of said comparison.

4. Method according to claim 2, whereby determining said activity of a signal transduction pathway involving PIK3CA comprises determining amplification of a gene encoding PIK3CA.

5. Method according to claim 2, whereby determining said activity of a signal transduction pathway involving PIK3CA comprises determining a level of expression of a gene product from the PIK3CA gene.

6. Method according to claim 2, whereby determining said activity of a signal transduction pathway involving PIK3CA comprises determining a kinase activity of PIK3CA.

7. A method according to claim 1 or claim 2, comprising determining a nucleotide sequence from PIK3CA at any of positions 1624, 1633, 1634, or 3140 of a nucleic acid encoding PIK3CA, as depicted in Figure 7, or of the corresponding position in a genomic nucleic acid molecule encoding PIK3CA, or of the corresponding position in any part or derivative of the indicated nucleotide sequence, whereby an alteration of the encoded amino acid at position 542 (glutamic acid), 545 (glutamic acid), and 1047 (histidine), from the indicated amino acid to another amino acid, is indicative of a risk of exhibiting or acquiring a reduced response towards HER2-targeting therapy.

8. Method according to claim 2, whereby determining an activity of a signal transduction pathway involving PIK3CA comprises determining a level of expression of a PIK3CA-indicator gene.

9. Method according to any of the previous claims, whereby said HER2-targeting therapy is selected from gene therapy, chemo-therapy, compound-mediated therapy, siRNA-mediated therapy, protein therapy, and antibody-mediated therapy.

10. Method according to claim 9, whereby said HER2-targeting therapy comprises antibody-mediated therapy.

11. Method according to claim 10, whereby said antibody-mediated therapy comprises trastuzumab.

12. Use of an inhibitor of PIK3CA, or an inhibitor of a mutant of PIK3CA, in the preparation of a medicament for the treatment of a HER2-resistant cancer patient.

13. Use according to claim 12, wherein said cancer patient is a breast cancer patient.


SEQUENCE LISTING

<110> Het Nederlands Kanker Instituut

<120> Involvement of lipid kinase, and signal transduction pathway
comprising said lipid kinase, in resistance to HER2-targeting
therapy

<130> P81046EP00

<140> EP 07106144.4
<141> 2007-04-13

<160> 8

<170> PatentIn version 3.3

<210> 1
<211> 3724
<212> DNA
<213> Homo sapiens

<220>
<221> CDS
<222> (158)..(3361)

<400> 1
tctccctcgg cgccgccgcc gccgcccgcg gggctgggac ccgatgcggt tagagccgcg 60
gagcctggaa gagccccgag cgtttctgct ttgggacaac catacatcta attccttaaa 120
gtagttttat atgtaaaact tgcaaagaat cagaaca atg cct cca cga cca tca 175
tca ggt gaa ctg tgg ggc atc cac ttg atg ccc cca aga atc cta gta 223
gaa tgt tta cta cca aat gga atg ata gtg act tta gaa tgc ctc cgt 271
gag gct aca tta ata acc ata aag cat gaa cta ttt aaa gaa gca aga 319
aaa tac ccc ctc cat caa ctt ctt caa gat gaa tct tct tac att ttc 367
gta agt gtt act caa gaa gca gaa agg gaa gaa ttt ttt gat gaa aca 415
aga cga ctt tgt gac ctt cgg ctt ttt caa ccc ttt tta aaa gta att 463
gaa cca gta ggc aac cgt gaa gaa aag atc ctc aat cga gaa att ggt 511
ttt gct atc ggc atg cca gtg tgt gaa ttt gat atg gtt aaa gat cca 559
gaa gta cag gac ttc cga aga aat att ctg aac gtt tgt aaa gaa gct 607
gtg gat ctt agg gac ctc aat tca cct cat agt aga gca atg tat gtc 655
tat cct cca aat gta gaa tct tca cca gaa ttg cca aag cac ata tat 703
aat aaa tta gat aaa ggg caa ata ata gtg gtg atc tgg gta ata gtt 751
tct cca aat aat gac aag cag aag tat act ctg aaa atc aac cat gac 799
tgt gta cca gaa caa gta att gct gaa gca atc agg aaa aaa act cga 847
agt atg ttg cta tcc tct gaa caa cta aaa ctc tgt gtt tta gaa tat 895
cag ggc aag tat att tta aaa gtg tgt gga tgt gat gaa tac ttc cta 943
gaa aaa tat cct ctg agt cag tat aag tat ata aga agc tgt ata atg 991
ctt ggg agg atg ccc aat ttg atg ttg atg gct aaa gaa agc ctt tat 1039
tct caa ctg cca atg gac tgt ttt aca atg cca tct tat tcc aga cgc 1087
att tcc aca gct aca cca tat atg aat gga gaa aca tct aca aaa tcc 1135
ctt tgg gtt ata aat agt gca ctc aga ata aaa att ctt tgt gca acc 1183
tac gtg aat gta aat att cga gac att gat aag atc tat gtt cga aca 1231
ggt atc tac cat gga gga gaa ccc tta tgt gac aat gtg aac act caa 1279
aga gta cct tgt tcc aat ccc agg tgg aat gaa tgg ctg aat tat gat 1327
ata tac att cct gat ctt cct cgt gct gct cga ctt tgc ctt tcc att 1375
tgc tct gtt aaa ggc cga aag ggt gct aaa gag gaa cac tgt cca ttg 1423
gca tgg gga aat ata aac ttg ttt gat tac aca gac act cta gta tct 1471
gga aaa atg gct ttg aat ctt tgg cca gta cct cat gga tta gaa gat 1519
ttg ctg aac cct att ggt gtt act gga tca aat cca aat aaa gaa act 1567
cca tgc tta gag ttg gag ttt gac tgg ttc agc agt gtg gta aag ttc 1615
cca gat atg tca gtg att gaa gag cat gcc aat tgg tct gta tcc cga 1663
gaa gca gga ttt agc tat tcc cac gca gga ctg agt aac aga cta gct 1711
aga gac aat gaa tta agg gaa aat gac aaa gaa cag ctc aaa gca att 1759
tct aca cga gat cct ctc tct gaa atc act gag cag gag aaa gat ttt 1807
cta tgg agt cac aga cac tat tgt gta act atc ccc gaa att cta ccc 1855
aaa ttg ctt ctg tct gtt aaa tgg aat tct aga gat gaa gta gcc cag 1903
atg tat tgc ttg gta aaa gat tgg cct cca atc aaa cct gaa cag gct 1951
atg gaa ctt ctg gac tgt aat tac cca gat cct atg gtt cga ggt ttt 1999
gct gtt cgg tgc ttg gaa aaa tat tta aca gat gac aaa ctt tct cag 2047
tat tta att cag cta gta cag gtc cta aaa tat gaa caa tat ttg gat 2095
aac ttg ctt gtg aga ttt tta ctg aag aaa gca ttg act aat caa agg 2143
att ggg cac ttt ttc ttt tgg cat tta aaa tct gag atg cac aat aaa 2191
aca gtt agc cag agg ttt ggc ctg ctt ttg gag tcc tat tgt cgt gca 2239
tgt ggg atg tat ttg aag cac ctg aat agg caa gtc gag gca atg gaa 2287
aag ctc att aac tta act gac att ctc aaa cag gag aag aag gat gaa 2335
aca caa aag gta cag atg aag ttt tta gtt gag caa atg agg cga cca 2383
gat ttc atg gat gct cta cag ggc ttt ctg tct cct cta aac cct gct 2431
cat caa cta gga aac ctc agg ctt gaa gag tgt cga att atg tcc tct 2479
gca aaa agg cca ctg tgg ttg aat tgg gag aac cca gac atc atg tca 2527
gag tta ctg ttt cag aac aat gag atc atc ttt aaa aat ggg gat gat 2575
tta cgg caa gat atg cta aca ctt caa att att cgt att atg gaa aat 2623
atc tgg caa aat caa ggt ctt gat ctt cga atg tta cct tat ggt tgt 2671
ctg tca atc ggt gac tgt gtg gga ctt att gag gtg gtg cga aat tct 2719
cac act att atg caa att cag tgc aaa ggc ggc ttg aaa ggt gca ctg 2767
cag ttc aac agc cac aca cta cat cag tgg ctc aaa gac aag aac aaa 2815
gga gaa ata tat gat gca gcc att gac ctg ttt aca cgt tca tgt gct 2863
gga tac tgt gta gct acc ttc att ttg gga att gga gat cgt cac aat 2911
agt aac atc atg gtg aaa gac gat gga caa ctg ttt cat ata gat ttt 2959
gga cac ttt ttg gat cac aag aag aaa aaa ttt ggt tat aaa cga gaa 3007
cgt gtg cca ttt gtt ttg aca cag gat ttc tta ata gtg att agt aaa     3055
gga gcc caa gaa tgc aca aag aca aga gaa ttt gag agg ttt cag gag     3103
atg tgt tac aag gct tat cta gct att cga cag cat gcc aat ctc ttc     3151
ata aat  ctt ttc tca atg atg  ctt ggc tct gga atg  cca gaa cta     3196
caa tct  ttt gat gac att gca  tac att cga aag acc  cta gcc tta     3241
gat aaa  act gag caa gag gct  ttg gag tat ttc atg  aaa caa atg     3286
aat gat  gca cat cat ggt ggc  tgg aca aca aaa atg  gat tgg atc     3331
ttc cac  aca att aaa cag cat  gca ttg aac tgaaaagata actgagaaaa     3381
tgaaagctca ctctggattc cacactgcac tgttaataac tctcagcagg caaagaccga     3441
ttgcatagga attgcacaat ccatgaacag cattagaatt tacagcaaga acagaaataa     3501
aatactatat aatttaaata atgtaaacgc aaacagggtt tgatagcact taaactagtt     3561
catttcaaaa ttaagcttta gaataatgcg caatttcatg ttatgcctta agtccaaaaa     3621
ggtaaacttt gaagattgtt tgtatctttt tttaaaaaac aaaacaaaac aaaaatcccc     3681
aaaatatata gaaatgatgg agaaggaaaa aaaaaaaaaa aaa                     3724


<210>  2
<211>  1068
<212>  PRT
<213>  Homo sapiens

<400>  2

<210>  3
<211>  62
<212>  DNA
<213>  Artificial

<220>
<223>  primer pRS-T7-fw

<400>  3
ggccagtgaa ttgtaatacg actcactata gggaggcggc ccttgaacct cctcgttcga      60

cc                                                                      62

<210>  4
<211>  20
<212>  DNA
<213>  Artificial

<220>

<223>  primer pRS-8-rev

<400>  4
taaagcgcat gctccagact                                                    20


<210>  5
<211>  24
<212>  DNA
<213>  Artificial

<220>
<223>  primer exon 9-fw

<400>  5
agtaacagac tagctagaga caat                                               24


<210>  6
<211>  25
<212>  DNA
<213>  Artificial

<220>
<223>  primer exon 9-rev

<400>  6
gagatcagcc aaattcagtt atttt                                              25


<210>  7
<211>  24
<212>  DNA
<213>  Artificial

<220>
<223>  primer exon 20-fw

<400>  7
caggagatgt gttacaaggc ttat                                               24


<210>  8
<211>  24
<212>  DNA
<213>  Artificial

<220>
<223>  primer exon 20-rev

<400>  8
tcagttcaat gcatgctgtt taat                                               24